# EUROPEAN PATENT SPECIFICATION

(11) **EP 3 797 964 B1**
(45) Date of publication and mention of the grant of the patent: **07.06.2023**
(21) Application number: 19199731.1
(22) Date of filing: 26.09.2019
(51) Int. Cl.: B29C 45/26, B29C 45/37, B29L 31/00, A61M 15/00, B29C 45/33

(54) **INJECTION MOLDING TOOL FOR METERED DOSE INHALER**
SPRITZGIESSWERKZEUG FÜR INHALATOR ABGEMESSENER DOSEN
OUTIL DE MOULAGE PAR INJECTION POUR INHALATEUR DE DOSE MESURÉE

(43) Date of publication of application: 31.03.2021
(73) Proprietor: Presspart Manufacturing S.A., 43720 L'Arboc del Penedes, Tarragona (ES)
(72) Inventor: Torrres Muniesa, Victor, E-08197 Sant Cugat del Vallès Barcelona (ES); Busto Sotil, Santiago, E-08730 Els Monjos Barcelona (ES); Roig Gilabert, Jordi, E-43141 Vilallonga del Camp Tarragona (ES); Herrera Martinez, Paloma, E-43820, Tarragona (ES)
(74) Representative: Uexküll & Stolberg

(56) References cited:
- GB-A- 1 326 714
- US-A- 2 783 502
- US-A- 2 962 228
- US-A1- 2016 101 244

## Description

### FIELD OF THE INVENTION

The present invention relates to the field of injection molding tools for nozzle blocks and metered dose inhalers with nozzle blocks. The invention also relates to an injection molding machine comprising a molding tool. Patent document US 2 783 502 A depicts an injection molding tool.

### BACKGROUND OF THE INVENTION

Metered dose inhalers, such as pressured metered dose inhalers (pMDI), are medication delivery devices which deliver a pharmaceutical formulation including one or more pharmaceutical active compounds to a human or another mammalian patient. Typically, the pharmaceutical formulation is delivered by the pMDIs in unit doses in the form of an aerosol. Each actuation of the MDIs delivers one unit dose. The unit dose is expelled by the MDIs and is taken into the body of the patient on inhalation via the nose or mouth. The pharmaceutical formulation is delivered to or via the respiratory tract, notably to the lungs, of the patient on inhalation. Metered dose inhalers are typically used for the treatment of respiratory infections and disorders, including respiratory tract infections, obstructive lung disease, inflammatory lung disease and chronic obstructive pulmonary disease. Asthma treatment is a particularly common use of MDIs.

The medicament is typically contained in a container capable of withstanding the vapor pressure of the propellant, such as plastic or plastic-coated glass bottle or preferably a metal can, for example a stainless steel can or aluminium can which may be anodised, organic coated, which container is closed with a metering valve. Metering valves comprising a metering chamber are designed to deliver a metered amount of the formulation per actuation. A valve stem extends from the metering valve and acts as a conduit to pass the metered dose into a nozzle block located in the inhaler body, in which the valve stem is seated. The nozzle block defines the walls of the valve stem receptacle, an expansion chamber (also defined as sump), and a nozzle channel which ends in an orifice and an enlarging frusto-conical section positioned adjacent to the orifice in streaming direction of the metered dose. The orifice and the frusto-conical section together form a nozzle of the nozzle block. The frusto-conical section of the nozzle is referred to hereinafter as bowl of the nozzle.

In order to ensure proper functioning of the inhalers it has to be ensured that in particular dimensions of the nozzle block - which hosts the expansion chamber and nozzle channel - lie within a given specification. This is because the design of the nozzle block significantly influences the spray pattern of the aerosol exiting the nozzle towards a second open end of the inhaler body, in particular the mouthpiece of the inhaler body. Within the nozzle block device variables that influence drug delivery from pressurized metered-dose inhalers are expansion chamber size and shape, orifice diameter of the nozzle and nozzle channel length (see S.P. Newman "Principles of Metered-Dose Inhaler Design", Respiratory Care, Vol. 50, No. 9 (September 2005), pages 1177-1190).

Before firing, a channel between the body of the container and the metering chamber is open, but as the MDI is fired, this channel closes, and another channel connecting the metering chamber to the atmosphere opens. The pressurized formulation is expelled rapidly into the nozzle block. When the dose leaves the nozzle, the liquid ligaments embedded in the propellant vapor are pulled apart by aerodynamic forces to form a dispersion of liquid droplets. Evaporation of propellant, both in the initial flashing and as the droplets move away from the nozzle, cools the droplets.

The design of the nozzle block is important, particularly because the aerosol particle size is determined partly by the orifice diameter of the nozzle. Aerosol particle size varies directly with orifice diameter, and particle size influences lung deposition. Aerosol particle size can also be modified by changing the length of the nozzle channel, hereinafter "jet length" (JL).

Conventional pressurized metered dose inhalers have different orifice diameters ranging from 0.25 to 0.42 mm and a jet length ranging from 0.30 to 1.7 mm. WO 0l/l9342 A1 discloses orifice diameters in the range of 0.15 to 0.45 mm, particularly 0.2 to 0.45 mm. According to this prior art reference it is advantageous to use a small orifice diameter e.g. 0.25 mm or less, particularly 0.22 mm since this tends to result in a higher FPM (fine particle mass) and lower throat deposition.

The nozzle block hosting the expansion chamber and nozzle channel, and often also the rest of the inhaler body, are usually injection moulded in one piece, as a single unit, through single injection molding tools. Alternatively, inhaler bodies may be moulded in one piece with exception of the nozzle channel, which is later laser drilled, such as described in WO 03/053501 A1, to enable even smaller orifice diameters.

As the inhaler body incorporates in its interior the nozzle block which in turn incorporates the valve stem receptacle, the expansion chamber, the nozzle and the nozzle channel, the injection molding tool in which the inhaler body is formed, consists of various tool components which define the cavity into which the inhaler body material is injected. At any time the design parameters of the nozzle channel, e.g. orifice diameter and/or jet length, is to be changed, it is necessary to disassemble the entire injection molding tool and to reassemble it using different parts.

For example, the jet length within the nozzle block is defined by a pin in the injection molding tool which protrudes from a tool section of a tool component. The tool section defines a cone angle of the nozzle. The tool section rests against another tool section which defines the expansion chamber within the nozzle block. In many instances it is possible to maintain the orifice diameter and change the jet length within the nozzle block when switching the manufacture of a metered dose inhaler from one medicament to another. Nevertheless, the injection molding tool will have to be disassembled completely, and reassembled containing a different tool which defines cone angle and jet length.

It is an object of the present invention to provide an injection molding tool for metered dose inhaler bodies with which the jet length in the nozzle block may be varied without having to disassemble and to reassemble the injection molding tool.

### SUMMARY OF THE INVENTION

The object is achieved with the injection molding tool as defined in present claim 1. Preferred embodiments are illustrated in the appending sub-claims.

According to the invention, there is provided an injection molding tool for the manufacture of at least a nozzle block of a metered dose inhaler body, the nozzle block having a valve stem receptacle, an expansion chamber and a nozzle, the nozzle being connected to the expansion chamber via a nozzle channel having a defined length (jet length). The injection molding tool comprises multiple tool components forming a cavity in which at least the nozzle block of the metered dose inhaler body is formed via injection molding. The tool components comprise a first tool section which defines the form of the valve stem receptacle and of the expansion chamber of the nozzle block, and a second tool section comprising a first portion for forming the nozzle and a second portion for forming the nozzle channel of the nozzle block. The second portion is formed as a pin protruding from the first portion towards the first tool section and the pin rests against the first tool section upon forming the nozzle block of the metered dose inhaler body via injection molding. The first portion of the second tool section and the pin are movable against each other for adjusting the jet length of the nozzle channel.

Preferably, based on the desired spray pattern and according to the given specifications regarding the dimensions of the nozzle block the optimal jet length for the individually inhaler body to be produced is defined and the injection molding tool for the manufacturer of at least a nozzle block is adjusted accordingly. In detail, the first portion of the second tool section and the pin are moved against each other such that the desired jet length of the nozzle channel is adjusted. Preferably, the adjustment of the desired jet length may be accomplished while the injection molding tool is mounted in the corresponding injection molding machine. This allows adjustment of the jet length of the nozzle channel without having to disassemble and reassemble the injection molding tool. Optionally, the nozzle channel is formed cylindrical such that the orifice diameter and the diameter of the nozzle channel are identical.

In an embodiment of the invention the second tool section comprises a biasing member for generating a biasing force acting onto the pin by which the pin is pushed against the first tool section. By pushing the pin against the first tool section it is ensured that the pin preferably fully abuts the first tool section and that no molded plastic material penetrates there between while the nozzle block is injection molded. This ensures proper forming of the transmission between the expansion chamber and the nozzle channel of the nozzle block.

In another embodiment of the invention, the basing member is a spring, wherein the spring and the pin are coaxially arranged. Preferably, the spring is a helical compression spring which is cost efficient and durable. Moreover, using a helical compression spring and arranging the spring coaxially with the pin minimizes the installation space needed.

Preferably, the first portion of the second tool section circumferentially surrounds the pin. Optionally, the pin is partly installed within the first portion of the second tool section which allows a space saving arrangement. Preferably, the first portion also circumferentially surrounds the biasing member.

In an embodiment of the invention the first portion of the second tool section comprises a through hole through which the pin extends and from which the pin protrudes. Optionally, the first portion, in particular the through hole, is configured to properly position the pin relative to the nozzle, in particular to the bowl of the nozzle.

In another embodiment of the invention the first portion of the second tool section comprises a front face arranged adjacent to the through hole wherein the front face is configured to define a nozzle exit angle (cone angle) of the nozzle of the nozzle block. Preferably, the distance within which the pin protrudes from the front face of the first portion defines the jet length of the nozzle block. As the first portion defines the form of the nozzle, in particular the nozzle exit angle, the position of the through hole provides a proper arrangement of the nozzle channel relative to the bowl of the nozzle.

Preferably, the second tool section comprises a driving unit for driving the first portion relative to the pin along a longitudinal axis of the pin in order to adjust the jet length of the nozzle channel. The driving unit allows easy adjustment of the jet length from outside of the injection molding tool. The injection molding tool does not have to be disassembled and reassembled again in order to adjust the jet length. The driving unit may comprise mechanical and/or electronic driving members which allow easy adjustment of the jet length. Preferably, when the jet length is to be adjusted and the pin rests against the first tool section, the pin remains static whereas the first portion of the second tool section is driven relative to the pin.

In an embodiment of the invention the driving unit is formed as a movable slider comprising at least one sliding surface, wherein upon movement of the slider a protrusion of the first portion of the second tool section slides along the sliding surface thereby moving the first portion relative to the pin. The use of a sliding surface which contacts a protrusion of the first portion of the second tool section provides an easy and cost efficient way to allow movement of the first portion relative to the pin.

In another embodiment of the invention the movable slider is movable in a direction transverse to the longitudinal axis of the pin. Preferably the injection molding tool comprises multiple plates which are stacked above each other. Arranging the movable slider relative to the longitudinal axis of the pin in a transverse direction allows a flat space saving construction.

In an embodiment of the invention the movable slider comprises two wedges each forming one sliding surface, wherein the sliding surfaces are arranged such that they oppose each other and wherein the protrusion of the first portion of the second tool section is arranged between the opposing sliding surfaces. By using two wedges each forming a sliding surface the first portion of the second tool section may be driven in opposing directions relative to the pin. Thus, the jet length of the nozzle channel may be shortened as well as lengthened depending on the sliding direction of the slider. Preferably, the slider may be moved back or forth.

Preferably, the first tool section extends along a first axis which runs centric through the valve stem receptacle towards the expansion chamber of the nozzle block and the second tool section extends along a second axis which runs centric though the pin towards the first tool section, wherein the first axis and the second axis enclose an angle of at least 90°, preferably of 90°-120°, more preferably of 95°-110°. Optionally, the nozzle channel and the nozzle are arranged such that they are aligned with the mouth piece of an inhaler body, whereas the valve stem receptacle is aligned with an opening of the inhaler body configured to receive a medicament container with a valve stem at its lower end. Preferably, the second axis is identical with the longitudinal axis of the pin of the first portion of the second tool section.

In an embodiment of the invention the first tool section and the second tool section are retractable from the cavity in which the nozzle block of the metered dose inhaler body is formed in order to eject the nozzle block from the injection molding tool. By using retractable tool sections undercuts may be formed by use of the injection molding tool.

In another embodiment of the invention the injection molding tool is configured to manufacture the metered dose inhaler body with the nozzle block, wherein the multiple tool components are configured to form a cavity in which the metered dose inhaler body with the nozzle block is formed via injection molding. Preferably, the metered dose inhaler body is formed together with the nozzle block at once. Optionally, the first tool section at least partially forms a boundary for the section of the metered dose inhaler body through which the aerosol container is inserted. Optionally, the second tool section forms a boundary for the mouth piece of the inhaler body.

In another embodiment the tool components comprise a third tool section which defines an opening in the metered dose inhaler body which is configured such that indica of a dose counter arranged inside the metered dose inhaler body are visible to a user through the opening, wherein the first tool section, the second tool section and the third tool section are retractable from the cavity in which the nozzle block of the metered dose inhaler body is formed in order to eject the nozzle block from the injection molding tool. Preferable, the third tool section rests against the first tool section upon forming the metered dose inhaler body of the metered dose inhaler via injection molding.

In connection with the present invention the term indica is to be understood as describing single numbers or signs located on a dose counting wheel of a mechanical dose counter in order to indicate the number of doses having been dispensed or remaining in the medicament container. In connection with the present invention the term indica is also to be understood as describing the numbers or signs displayed on an electronic display of an electronic dose counter in order to indicate the number of doses having been dispensed or remaining in the medicament container.

The above object is also achieved with an injection molding machine comprising an injection molding tool as described above.

The injection molding machine according to the invention allows easy adjustment of the jet length of the nozzle block to be produced via injection molding without having to disassemble and reassemble the injection molding tool.

### BRIEF DESCRIPTION OF THE FIGURES

The invention will now be described in connection with an exemplary embodiment shown in the Figures in which:
- Figure 1: shows a cross-sectional view of a metered dose inhaler body with a nozzle block in detail;
- Figure 2: shows a cross-sectional view of an injection molding tool with a first, a second and a third tool section; and
- Figure 3: shows a cross-sectional view of the second tool section of Figure 2 in detail.

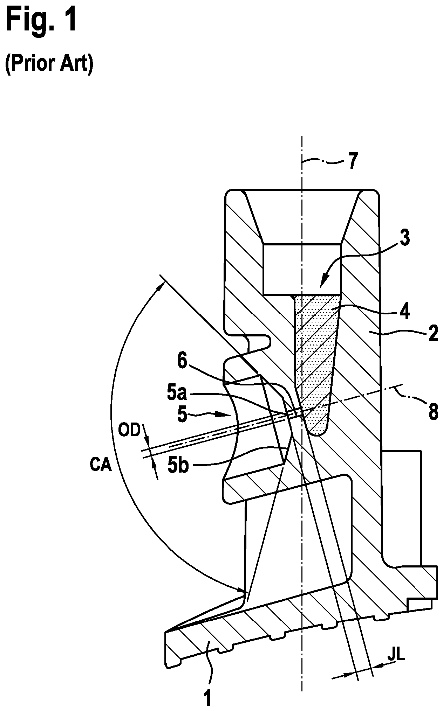

Figure 1 shows a partial view of a metered dose inhaler body 1 with a nozzle block 2 known in the art. The nozzle block 2 comprises a valve stem receptacle 3 which is configured to receive the valve stem of an aerosol container (not shown) inserted in the metered dose inhaler body 1. The nozzle block 2 further comprises an expansion chamber 4, a nozzle 5 and a nozzle channel 6 connecting the expansion chamber 4 with the nozzle 5. The nozzle channel 6 has a defined length which is referred to herein after as jet length (JL). The nozzle 5 comprises an orifice 5a and an enlarging frusto-conical section adjacent to the orifice, the frusto-conical section being referred to hereinafter as bowl 5b of the nozzle 5. The orifice 5a has a diameter which is referred to herein after as orifice diameter (OD). As the nozzle channel 6 is cylindrical the orifice diameter (OD) is identical with the diameter of the nozzle channel 6.

Figure 1 also shows is a first axis 7 which runs centric through the valve stem receptacle 3 towards the expansion chamber 4 of the nozzle block 2 and a second axis 8 which runs centric through the nozzle channel 6. The first axis 7 and the second axis 8 enclosed angle of at least 90°, preferably of 90°-120°, more preferably of 95°-110°.

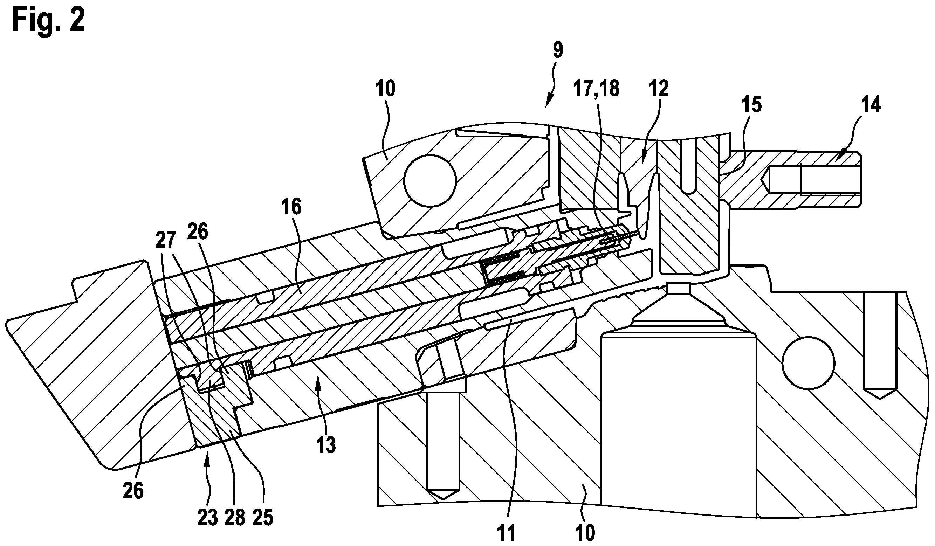

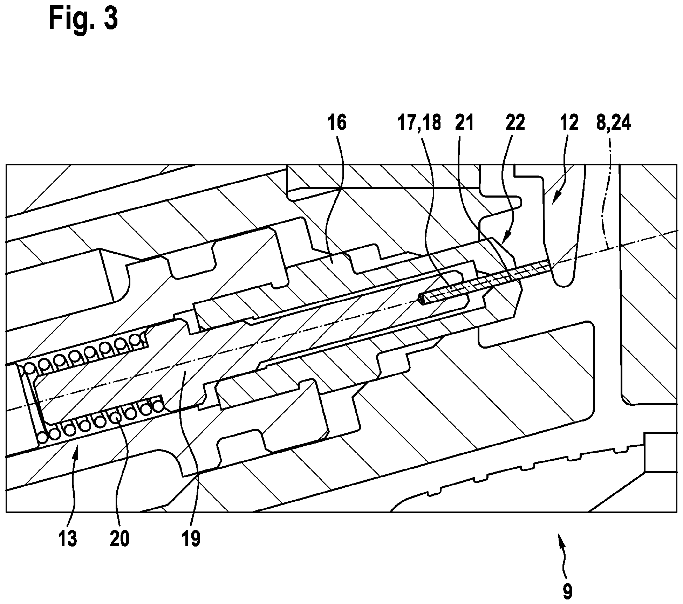

Figures 2 and 3 show an injection molding tool 9 which is used in an injection molding machine (not shown) in order to manufacture the metered dose inhaler body 1 (Fig. 1) with the nozzle block via injection molding. The injection molding tool 9 comprises multiple tool components 10 forming a cavity 11 in which the metered dose inhaler body 1 with the nozzle block 2 is formed.

The tool components 10 comprise a first tool section 12, a second tool section 13 and a third tool section 14. The first tool section 12, the second tool section 13 and the third tool section 14 are retractable from the cavity 11 in which the meter dose inhaler body 1 with the nozzle block 2 is formed in order to eject the metered dose inhaler body 1 with the nozzle block 2 from the injection molding tool 9. The first tool section 12 extends along the first axis 7 (Figure 1) and the second tool section 12 extends along the second axis 8 (Figure 1).

The first tool section 12 is configured to define the form of the valve stem receptacle 3 and of the expansion chamber 4 of the nozzle block 2. The third tool section 14 is configured to define an opening 15 in the metered dose inhaler body 1 which is configured such that indica of a dose counter (not shown) arranged inside the metered dose inhaler body 1 are visible to a user through the opening 15.

The second tool section 13 comprises a first portion 16 for forming the nozzle 5 and a second portion 17 for forming the nozzle channel 6 of the nozzle block 2. The second portion 17 is formed as a pin 18 protruding from the first portion 16 towards the first tool section 12. The pin 18 rests against the first tool section 12 upon forming the metered dose inhaler body 1 via injection molding.

The second tool section 13 comprises an intermediate member 19, which holds the pin 18. Moreover, the second tool section 13 comprises a biasing member formed as a spring 20 for generating a biasing force acting onto the pin 18 via the intermediate member 19. The pin 18 is pushed by the biasing force and the intermediate member 19 against the first tool section 12. The spring 20, the intermediate member 19 and the pin 18 are co-axially arranged. The first portion 16 of the second tool section 13 circumferentially surrounds the pin 18 and the intermediate member 19.

The first portion 16 of the second tool section 13 comprises a through hole 21 through which the pin 18 extends and from which the pin 18 protrudes. The first portion 16 comprises a front face 22 arranged adjacent to the through hole 21, wherein the front face 22 is configured to define the cone angle (CA) of the nozzle 5 of the nozzle block 2 (see Fig. 1).

The second tool section 13 further comprises a driving unit 23 for driving the first portion 16 relative to the pin 18 along a longitudinal axis 24 of the pin 18 in order to adjust the jet length (JL) of the nozzle channel 6. In other words: The first portion 16 of the second tool section 13 and the pin 18 are movable against each other for adjusting the jet length (JL) of the nozzle channel 6. The longitudinal axis 24 of the pin 18 is identical to the second axis 8 along which the second tool section 12 extends.

The driving unit 23 is formed as a movable slider 25 which is movable in a direction transverse to the longitudinal axis 24 of the pin 18. The slider 25 comprises two wedges 26 each forming one sliding surface 27, wherein the siding surfaces 27 are arranged such that they oppose each other. The first portion 16 of the second tool section 13 comprises a protrusion 28 which is arranged between the opposing sliding surfaces 27. The slider 25 is configured such that upon movement of the slider 25 the protrusion 28 of the first portion 16 slides along the sliding surfaces 27 thereby moving the first portion 16 relative to the pin 18. The slider 25 may be driven electronically by an electric motor or may be driven manually.

In the following the function of the injection molding tool 9 shall be explained with reference to Figures 1-3.

In order to adjust the jet length (JL) of the nozzle channel 6 the movable slider 25 is moved transverse to the longitudinal axis 24 of the pin 18. The protrusion 28 of the first portion 16 of the second tool section 13 slides along the sliding surfaces 27 of the slider 25 thereby moving the first portion 16 relative to the pin 18. As the spring 20 pushes the pin 18 against the first tool section 12 the pin 18 remains static whereas the first portion 16 is moved relative to the pin 18. By using the injection molding tool 9 according to the present invention the jet length (JL) may be shortened or lengthened by moving the slider 25 back and forth, respectively.

### Reference Numerals

- 1: Metered dose inhaler body
- 2: Nozzle block
- 3: Valve stem receptacle
- 4: Expansion chamber
- 5: Nozzle
- 5a: Orifice (nozzle)
- 5b: Bowl (nozzle)
- 6: Nozzle channel
- JL: Jet length
- CA: Cone angle
- 7: First axis
- 8: Second axis
- 9: Injection molding tool
- 10: Tool components
- 11: Cavity
- 12: First tool section
- 13: Second tool section
- 14: Third tool section
- 15: Opening (inhaler body)
- 16: First portion (second tool section)
- 17: Second portion (second tool section)
- 18: Pin (second portion)
- 19: Intermediate member (second tool section)
- 20: Spring
- 21: Through hole (first portion)
- 22: Front face (first portion)
- 23: Driving unit
- 24: Longitudinal axis
- 25: Slider
- 26: Wedges (slider)
- 27: Sliding surfaces (slider)
- 28: Protrusion (first portion)

## Claims

1. Injection molding tool for the manufacture of at least a nozzle block (2) of a metered dose inhaler body (1), the nozzle block (2) having a valve stem receptacle (3), an expansion chamber (4) and a nozzle (5), the nozzle (5) being connected to the expansion chamber (4) via a nozzle channel (6) having a defined length (jet length),
wherein the injection molding tool (9) comprises multiple tool components (10) forming a cavity (11) in which at least the nozzle block (2) of the metered dose inhaler body (1) is formed via injection molding, the tool components (10) comprising:
- a first tool section (12) which defines the form of the valve stem receptacle (3) and of the expansion chamber (4) of the nozzle block (2), and
- a second tool section (13) comprising a first portion (16) for forming the nozzle (5) and a second portion (17) for forming the nozzle channel (6) of the nozzle block (2),
wherein the second portion (17) is formed as a pin (18) protruding from the first portion (16) towards the first tool section (12) and,
wherein the pin (18) rests against the first tool section (12) upon forming the nozzle block (2) of the metered dose inhaler body (1) via injection molding,
**characterized in**
**that** the first portion (16) of the second tool section (13) and the pin (18) are movable against each other for adjusting the jet length of the nozzle channel (6) and, that the second tool section (13) comprises a driving unit (23) for driving the first portion (16) relative to the pin (18) along a longitudinal axis (24) of the pin (18) in order to adjust the jet length of the nozzle channel (6).

2. Injection molding tool according to claim 1, **characterized in that** that the second tool section (13) comprises a biasing member (20) for generating a biasing force acting onto the pin (18) by which the pin (18) is pushed against the first tool section (12).

3. Injection molding tool according to claim 2, **characterized in that** the biasing member is a spring (20), wherein the spring (20) and the pin (18) are coaxially arranged.

4. Injection molding tool according to any of the preceding claims, **characterized in that** the first portion (16) of the second tool section (13) circumferentially surrounds the pin (18).

5. Injection molding tool according to any of the preceding claims, **characterized in that** the first portion (16) of the second tool section (13) comprises a through hole (21) through which the pin (18) extends and from which the pin (18) protrudes.

6. Injection molding tool according to claim 5, **characterized in that** the first portion (16) of the second tool section (13) comprises a front face (22) arranged adjacent to the through hole (21) wherein the front face (22) is configured to define a nozzle exit angle (cone angle) of the nozzle (5) of the nozzle block (2).

7. Injection molding tool according to claim 1, **characterized in that** the driving unit (23) is formed as a movable slider (25) comprising at least one sliding surface (27), wherein upon movement of the slider (25) a protrusion (28) of the first portion (16) of the second tool section (13) slides along the sliding surface (27) thereby moving the first portion (16) relative to the pin (18).

8. Injection molding tool according to claim 7, **characterized in that** the movable slider (25) is movable in a direction transverse to the longitudinal axis (24) of the pin (18).

9. Injection molding tool according to claim 7, **characterized in that** the movable slider (25) comprises two wedges (26) each forming one sliding surface (27), wherein the sliding surfaces (27) are arranged such that they oppose each other and, wherein the protrusion (28) of the first portion (16) of the second tool section (13) is arranged between the opposing sliding surfaces (27).

10. Injection molding tool according to any of the preceding claims, **characterized in that** the first tool section (12) extends along a first axis (7) which runs centric through the valve stem receptacle (3) towards the expansion chamber (4) of the nozzle block (2) and that the second tool section (13) extends along a second axis (8) which runs centric through the pin (18) towards the first tool section (12), wherein the first axis (7) and the second axis (8) enclose an angle of at least 90°, preferably of 90° to 120°, more preferably of 95° to 110°.

11. Injection molding tool according to any of the preceding claims, **characterized in that** the first tool section (12) and the second tool section (13) are retractable from the cavity (11) in which the nozzle block (2) of the metered dose inhaler body (1) is formed in order to eject the nozzle block (2) from the injection molding tool (9).

12. Injection molding tool according to any of the preceding claims, **characterized in that** the injection molding tool (9) is configured to manufacture the metered dose inhaler body (1) with the nozzle block (2), wherein the multiple tool components (10) are configured to form a cavity (11) in which the metered dose inhaler body (1) with the nozzle block (2) is formed via injection molding.

13. Injection molding tool according to any of the preceding claims, **characterized in that** the tool components (10) comprise a third tool section (14) which defines an opening (15) in the metered dose inhaler body (1) which is configured such that indica of a dose counter arranged inside the metered dose inhaler body (1) are visible to a user through the opening (15), wherein the first tool section (12), the second tool section (13) and the third tool section (14) are retractable from the cavity (11) in which the nozzle block (2) of the metered dose inhaler body (1) is formed in order to eject the nozzle block (2) from the injection molding tool (9).

14. Injection molding machine comprising an injection molding tool (9) according to any of the preceding claims 1 to 13.

## Patentansprüche

1. Spritzgießwerkzeug zur Herstellung mindestens eines Düsenblocks (2) eines Dosierinhalatorkörpers (1), wobei der Düsenblock (2) eine Ventilschaftaufnahme (3), eine Expansionskammer (4) und eine Düse (5) aufweist, wobei die Düse (5) mit der Expansionskammer (4) über einen Düsenkanal (6) mit einer definierten Länge (Strahllänge) verbunden ist, wobei das Spritzgießwerkzeug (9) mehrere Werkzeugkomponenten (10) umfasst, die einen Hohlraum (11) bilden, in dem zumindest der Düsenblock (2) des Dosierinhalatorkörpers (1) durch Spritzgießen gebildet wird, wobei die Werkzeugkomponenten (10) umfassen:
- einen ersten Werkzeugabschnitt (12), der die Form der Ventilschaftaufnahme (3) und der Expansionskammer (4) des Düsenstocks (2) definiert, und
- einen zweiten Werkzeugabschnitt (13) mit einem ersten Teil (16) zur Bildung der Düse (5) und einem zweiten Teil (17) zur Bildung des Düsenkanals (6) des Düsenblocks (2),
wobei der zweite Teil (17) als ein Stift (18) ausgebildet ist, der von dem ersten Teil (16) in Richtung des ersten Werkzeugabschnitts (12) vorsteht und,
wobei der Stift (18) beim Formen des Düsenblocks (2) des Dosierinhalatorkörpers (1) durch Spritzgießen an dem ersten Werkzeugabschnitt (12) anliegt,
**dadurch gekennzeichnet,**
**dass** der erste Teil (16) des zweiten Werkzeugabschnitts (13) und der Stift (18) gegeneinander beweglich sind, um die Strahllänge des Düsenkanals (6) einzustellen und,
**dass** der zweite Werkzeugabschnitt (13) eine Antriebseinheit (23) zum Antreiben des ersten Teils (16) relativ zu dem Stift (18) entlang einer Längsachse (24) des Stifts (18) umfasst, um die Strahllänge des Düsenkanals (6) einzustellen.

2. Spritzgießwerkzeug nach Anspruch 1, **dadurch gekennzeichnet, dass** der zweite Werkzeugabschnitt (13) ein Vorspannelement (20) zur Erzeugung einer auf den Stift (18) wirkenden Vorspannkraft aufweist, durch die der Stift (18) gegen den ersten Werkzeugabschnitt (12) gedrückt wird.

3. Spritzgießwerkzeug nach Anspruch 2, **dadurch gekennzeichnet, dass** das Vorspannelement eine Feder (20) ist, wobei die Feder (20) und der Stift (18) koaxial angeordnet sind.

4. Spritzgießwerkzeug nach einem der vorhergehenden Ansprüche, **dadurch gekennzeichnet, dass** der erste Teil (16) des zweiten Werkzeugabschnitts (13) den Stift (18) umfangsmäßig umgibt.

5. Spritzgießwerkzeug nach einem der vorhergehenden Ansprüche, **dadurch gekennzeichnet, dass** der erste Teil (16) des zweiten Werkzeugabschnitts (13) ein Durchgangsloch (21) aufweist, durch das sich der Stift (18) erstreckt und aus dem der Stift (18) herausragt.

6. Spritzgießwerkzeug nach Anspruch 5, **dadurch gekennzeichnet, dass** der erste Teil (16) des zweiten Werkzeugabschnitts (13) eine Stirnfläche (22) umfasst, die neben dem Durchgangsloch (21) angeordnet ist, wobei die Stirnfläche (22) konfiguriert ist, einen Düsenaustrittswinkel (Kegelwinkel) der Düse (5) des Düsenblocks (2) zu definiert.

7. Spritzgießwerkzeug nach Anspruch 1, **dadurch gekennzeichnet, dass** die Antriebseinheit (23) als beweglicher Schieber (25) ausgebildet ist, der mindestens eine Gleitfläche (27) aufweist, wobei bei Bewegung des Schiebers (25) ein Vorsprung (28) des ersten Teils (16) des zweiten Werkzeugabschnitts (13) entlang der Gleitfläche (27) gleitet, wodurch der erste Teil (16) relativ zu dem Stift (18) bewegt wird.

8. Spritzgießwerkzeug nach Anspruch 7, **dadurch gekennzeichnet, dass** der bewegliche Schieber (25) in einer Richtung quer zur Längsachse (24) des Stiftes (18) bewegbar ist.

9. Spritzgießwerkzeug nach Anspruch 7, **dadurch gekennzeichnet, dass** der bewegliche Schieber (25) zwei Keile (26) umfasst, die jeweils eine Gleitfläche (27) bilden, wobei die Gleitflächen (27) so angeordnet sind, dass sie einander gegenüberliegen, und wobei der Vorsprung (28) des ersten Teils (16) des zweiten Werkzeugabschnitts (13) zwischen den gegenüberliegenden Gleitflächen (27) angeordnet ist.

10. Spritzgießwerkzeug nach einem der vorhergehenden Ansprüche, **dadurch gekennzeichnet, dass** sich der erste Werkzeugabschnitt (12) entlang einer ersten Achse (7) erstreckt, die zentrisch durch die Ventilschaftaufnahme (3) zur Expansionskammer (4) des Düsenstocks (2) verläuft und dass sich der zweite Werkzeugabschnitt (13) entlang einer zweiten Achse (8) erstreckt, die zentrisch durch den Stift (18) zum ersten Werkzeugabschnitt (12) verläuft, wobei die erste Achse (7) und die zweite Achse (8) einen Winkel von mindestens 90°, vorzugsweise von 90° bis 120°, besonders bevorzugt von 95° bis 110°, einschließen.

11. Spritzgießwerkzeug nach einem der vorhergehenden Ansprüche, **dadurch gekennzeichnet, dass** der erste Werkzeugabschnitt (12) und der zweite Werkzeugabschnitt (13) aus dem Hohlraum (11), in dem der Düsenblock (2) des Dosierinhalatorkörpers (1) gebildet wird, zurückziehbar sind, um den Düsenblock (2) aus dem Spritzgießwerkzeug (9) auszuwerfen.

12. Spritzgießwerkzeug nach einem der vorhergehenden Ansprüche, **dadurch gekennzeichnet, dass** das Spritzgießwerkzeug (9) ausgebildet ist, den Dosierinhalatorkörpers (1) mit dem Düsenblock (2) herszustellen, wobei die mehreren Werkzeugkomponenten (10) ausgebildet sind, einen Hohlraum (11) zu bilden, in dem der Dosierinhalatorkörper (1) mit dem Düsenblock (2) durch Spritzgießen gebildet wird.

13. Spritzgießwerkzeug nach einem der vorhergehenden Ansprüche, **dadurch gekennzeichnet, dass** die Werkzeugkomponenten (10) einen dritten Werkzeugabschnitt (14) umfassen, der eine Öffnung (15) in dem Dosierinhalatorkörper (1) definiert, die so gestaltet ist, dass durch die Öffnung (15) Indizes eines im Inneren des Dosierinhalatorkörpers (1) angeordneten Dosiszählers für einen Benutzer sichtbar sind, wobei der erste Werkzeugabschnitt (12), der zweite Werkzeugabschnitt (13) und der dritte Werkzeugabschnitt (14) aus dem Hohlraum (11), in dem der Düsenblock (2) des Dosierinhalatorkörpers (1) gebildet wird, zurückziehbar sind, um den Düsenblock (2) aus dem Spritzgießwerkzeug (9) auszuwerfen.

14. Spritzgießmaschine umfassend ein Spritzgießwerkzeug (9) nach einem der vorhergehenden Ansprüche 1 bis 13.

## Revendications

1. Outil de moulage par injection destiné à la fabrication d'au moins un bloc de buse (2) d'un corps d'inhalateur de dose mesurée (1), le bloc de buse (2) présentant un logement pour tige de valve (3), une chambre d'expansion (4) et une buse (5), la buse (5) étant reliée à la chambre d'expansion (4) par un canal de buse (6) ayant une longueur définie (longueur de jet),
dans lequel l'outil de moulage par injection (9) comprend plusieurs composants d'outil (10) formant une cavité (11) dans laquelle au moins le bloc de buse (2) du corps d'inhalateur de dose mesurée (1) est formé par moulage par injection, les composants d'outil (10) comprenant :
- une première partie d'outil (12) qui définit la forme du logement pour tige de valve (3) et de la chambre d'expansion (4) du bloc de buse (2), et
- une deuxième partie d'outil (13) comprenant une première portion (16), destinée à former la buse (5), et une deuxième portion (17) destinée à former le canal de buse (6) du bloc de buse (2),
où la deuxième portion (17) est réalisée sous la forme d'une tige (18) faisant saillie depuis la première portion (16) en direction de la première partie d'outil (12), et
où la tige (18) est en appui contre la première partie d'outil (12) après la réalisation du bloc de buse (2) du corps d'inhalateur de dose mesurée (1), par moulage par injection,
**caractérisé en ce que**
la première portion (16) de la deuxième partie d'outil (13) et la tige (18) peuvent être déplacées l'une par rapport à l'autre aux fins de régler la longueur de jet du canal de buse (6), et
**en ce que** la deuxième partie d'outil (13) comprend une unité d'entraînement (23) destinée à entraîner la première portion (16) par rapport à la tige (18), suivant un axe longitudinal (24) de la tige (18), afin de régler la longueur de jet du canal de buse (6).

2. Outil de moulage par injection selon la revendication 1, **caractérisé en ce que** la deuxième partie d'outil (13) comprend un élément de précontrainte (20) destiné à produire une force de précontrainte qui agit sur la tige (18) et par laquelle la tige (18) est poussée contre la première partie d'outil (12).

3. Outil de moulage par injection selon la revendication 2, **caractérisé en ce que** l'élément de précontrainte est un ressort (20), le ressort (20) et la tige (18) étant disposés de manière coaxiale.

4. Outil de moulage par injection selon l'une des revendications précédentes, **caractérisé en ce que** la première portion (16) de la deuxième partie d'outil (13) entoure la tige (18) sur la circonférence.

5. Outil de moulage par injection selon l'une des revendications précédentes, **caractérisé en ce que** la première portion (16) de la deuxième partie d'outil (13) présente un trou débouchant (21) à travers lequel s'étend la tige (18) et à partir duquel la tige (18) fait saillie.

6. Outil de moulage par injection selon la revendication 5, **caractérisé en ce que** la première portion (16) de la deuxième partie d'outil (13) présente une face frontale (22) disposée de façon adjacente au trou débouchant (21), la face frontale (22) étant configurée pour définir un angle de sortie de buse (angle de cône) de la buse (5) du bloc de buse (2).

7. Outil de moulage par injection selon la revendication 1, **caractérisé en ce que** l'unité d'entraînement (23) est réalisée sous la forme d'un curseur (25) mobile présentant au moins une surface de glissement (27), sachant que suite au mouvement du curseur (25), une saillie (28) de la première portion (16) de la deuxième partie d'outil (13) glisse le long de la surface de glissement (27), en déplaçant ainsi la première portion (16) par rapport à la tige (18).

8. Outil de moulage par injection selon la revendication 7, **caractérisé en ce que** le curseur (25) mobile peut être déplacé dans une direction transversale à l'axe longitudinal (24) de la tige (18).

9. Outil de moulage par injection selon la revendication 7, **caractérisé en ce que** le curseur (25) mobile comporte deux cales (26) qui forment chacune une surface de glissement (27), les surfaces de glissement (27) étant disposées de manière à ce qu'elles soient opposées l'une à l'autre, la saillie (28) de la première portion (16) de la deuxième partie d'outil (13) étant disposée entre les surfaces de glissement (27) opposées.

10. Outil de moulage par injection selon l'une des revendications précédentes, **caractérisé en ce que** la première partie d'outil (12) s'étend le long d'un premier axe (7) qui s'étend de manière centrée à travers le logement pour tige de valve (3), en direction de la chambre d'expansion (4) du bloc de buse (2), et **en ce que** la deuxième partie d'outil (13) s'étend le long d'un deuxième axe (8) qui s'étend de manière centrée à travers la tige (18), en direction de la première partie d'outil (12), le premier axe (7) et le deuxième axe (8) délimitant un angle d'au moins 90°, de préférence compris entre 90° et 120°, et de manière plus avantageuse compris entre 95° et 110°.

11. Outil de moulage par injection selon l'une des revendications précédentes, **caractérisé en ce que** la première partie d'outil (12) et la deuxième partie d'outil (13) peuvent être retirées de la cavité (11) dans laquelle le bloc de buse (2) du corps d'inhalateur de dose mesurée (1) est formé, aux fins d'éjecter le bloc de buse (2) de l'outil de moulage par injection (9).

12. Outil de moulage par injection selon l'une des revendications précédentes, **caractérisé en ce que** l'outil de moulage par injection (9) est configuré pour fabriquer le corps d'inhalateur de dose mesurée (1) avec le bloc de buse (2), les composants d'outil (10) multiples étant configurés pour former une cavité (11) dans laquelle le corps d'inhalateur de dose mesurée (1), avec le bloc de buse (2), est réalisé par moulage par injection.

13. Outil de moulage par injection selon l'une des revendications précédentes, **caractérisé en ce que** les composants d'outil (10) comprennent une troisième partie d'outil (14) qui définit une ouverture (15) dans le corps d'inhalateur de dose mesurée (1), qui est configurée de manière à ce que des indications d'un compteur de dose disposé à l'intérieur du corps d'inhalateur de dose mesurée (1) soient visibles pour un utilisateur à travers l'ouverture (15), la première partie d'outil (12), la deuxième partie d'outil (13) et la troisième partie d'outil (14) pouvant être retirées de la cavité (11) dans laquelle est formé le bloc de buse (2) du corps d'inhalateur de dose mesurée (1), aux fins d'éjecter le bloc de buse (2) de l'outil de moulage par injection (9).

14. Machine de moulage par injection comprenant un outil de moulage par injection (9) selon l'une des revendications précédentes 1 à 13.
